# EUROPÄISCHE PATENTANMELDUNG

(11) **EP 0 890 348 A1**
(43) Veröffentlichungstag der Anmeldung: **13.01.1999**
(21) Anmeldenummer: 97111777.5
(22) Anmeldetag: 10.07.1997
(51) Int. Cl.: A61F 5/41, A61N 2/02

(54) **Gerät zur Behandlung von Erektionsproblemen**

(71) Anmelder: Medionic GmbH, 90613 Grosshabersdorf (DE)
(72) Erfinder: Fischer, Horst, 90556 Seukendorf (DE)
(74) Vertreter: Bunse, Günter, Dr.

(57) **Zusammenfassung**

Gegenstand der Erfindung ist ein Gerät, daß die natürlichen, geschlechtsspezifischen physiologischen Funktionen des Mannes zu erhalten bzw. wiederherzustellen vermag.

Das Gerät besteht aus einer ringförmigen Magnetspule 1, die allseitig von einem Schaumstoffkörper 2 umhüllt ist, und zwar von einem äußeren und einem inneren Schaumstoffring 3 bzw. 4, wobei der erstere aus einer Schaumstoffschicht von einheitlicher Porengröße und der letztere aus zwei Schaumstoffschichten verschiedener Porengröße besteht.

Der Schaumstoffkörper weist oben und unten jeweils eine ringförmige Kappe 7 und 8 als Abdeckung auf, deren Ränder 10,12 nach innen umgebogen sind.

Die nach innen zu liegende Schaumstoffschicht des inneren Schaumstoffringes steht gegenüber den umgebogenen inneren Rändern der Abdeckung teilweise über.

Das von der Mägnetspule erzeugte (elektro-)magnetische Wechselfeld hat eine Leistung von bis zu 30 Gauß.

Die kreisrunde, für die Aufnahme des Penis vorgesehene Öffnung im Zentrum hat einen Durchmesser von etwa 4 cm.

Das Gerät ist so dimensioniert, daß es mit einer Hand umfaßt werden kann.

Durch regelmäßige Benutzung des Gerätes gelingt es, die natürlichen, geschlechtsspezifischen physiologischen Funktionen bis ins hohe Alter zu erhalten bzw. diese wiederherzustellen, was sich dadurch äußert, daß der Penis seine Erektionsfähigkeit weitgehend wiedererlangt.

Die Figur stellt einen Querschnitt des Gerätes dar.

## Beschreibung

Gegenstand der vorliegenden Erfindung ist ein Gerät, das die natürlichen, geschlechtsspezifischen physiologischen Funktionen des Mannes zu erhalten bzw. wiederherzustellen vermag.

Mit dem Älterwerden geht bei vielen Männern ein Nachlassen der natürlichen, geschlechtsspezifischen physiologischen Funktionen einher.

Dieses Nachlassen tritt insbesondere dadurch in Erscheinung, daß der Penis mehr oder weniger seine Fähigkeit zur Erektion einbüßt.

Mit dieser Einbuße ist nicht etwa diejenige gemeint, die krankheitsbedingt ist und auf einer Beeinträchtigung der Durchblutung oder auf einer Störung der vegetativen Funktionen zurückzuführen ist. Gemeint ist hier vielmehr ein Erschlaffen der natürlichen Reaktionen und die mit fortschreitendem Alter zunehmend auftretende Tonusschwäche, die in manchen Fällen dazu führen kann, daß die Fähigkeit zur Erektion vollständig verlorengeht.

Wenn man bedenkt, daß dieser Zustand in vielen Fällen im Alter ab 60 Jahren eintritt, in manchen Fällen schon ab 50 Jahren oder sogar noch früher, also in den sog. besten Mannesjahren, so erhellt daraus, daß mit diesem Zustand insbesondere Probleme psychologischer Art, wie die Beeinträchtigung oder der Verlust des Selbstwertgefühles, verknüpft sind oder jedenfalls verknüpft sein können.

Es hat daher nicht an Versuchen gefehlt, dieses Manko des Mannes zu beseitigen oder zumindest zu bessern.

Allen diesen Bemühungen ist bisher aber kein rechter Erfolg beschieden gewesen. So hat man beispielsweise durch einen operativen Eingriff den Penis mit einer elastischen Prothese bzw. Manschette aus gummi- oder kautschukartigem Material versehen, um auf diese Weise den Penis zu "stabilisieren", indem man eine natürliche Erektion vortäuscht. Es versteht sich, daß diese Methode keine Lösung des anstehenden Problems sein koste und nicht mehr als ein Notbehelf war.

Man ist auch mit therapeutischen Mitteln dem Problem zu Leibe gerückt, indem man die Impotenz oder zumindest die Potenzschwäche mit (Sexual-)Hormonen behandelt hat, selten allerdings mit dem gewünschten Erfolg. Der Penis blieb in der Regel schwach, knickte weg bzw. ein oder fiel im entscheidenden Augenbklick in sich zusammen. Je größer die Enttäuschung und je verbissener der Wille war, umso schlimmer wurde es.

Geradezu wie eine Erlösung haben es daher die Betroffenen empfunden, als die Methode der Selbstinjektion gefunden wurde, die als sog. Schwellkörper-Injektions-Therapie bekannt geworden ist. Es handelt sich dabei allerdings um eine Methode, die der ärztlichen Überwachung bzw. Kontrolle bedarf und die vor allem für Kranke entwickelt wurde und für solche Kranke bestimt ist, die blutdrucksenkende Medikamente einnehmen (müssen), wie Diabetiker oder Hypertoniker; denn es gilt die Regel, daß alles das, was den Blutdruck senkt, auch den Penis senkt.

Überraschenderweise wurde nun gefunden, daß man auf geradezu verblüffend einfache Art und Weise mit diesem Dilemma fertigwerden kann durch äußeres Anlegen eines (elektro-)magnetischen Wechselfeldes.

Gegenstand der Erfindung ist somit ein Gerät zur Erhaltung bzw. Wiederherstellung der natürlichen, geschlechtsspezischen physiologischen Funktionen des Mannes.

Das neue Gerät ist dadurch gekennzeichnet, daß es besteht aus einer ringförmigen Magnetspule 1, die allseitig von einem Schaumstoffkörper 2 umhüllt ist, und zwar von einem - von unten nach oben durchgehenden - äußeren, aus einer Schaumstoffschicht mit einheitlicher Porendichte bestehenden Schaumstoffring 3 und von einem - von unten nach oben durchgehenden -inneren, aus zwei Schaumstoffschichten verschiedener Porendichte bestehenden Schaumstoffring 4, bei dem die nach innen Zu liegende Schaumstoffschicht 5 gegenüber der anderen Schaumstoffschicht 6 eine größere Porendichte aufweist, daß der die Magnetspule umhüllende Schaumstoffkörper 2 am unteren und oberen Ende jeweils eine ringförmige, kappenartige Abdeckung aus Kunststoff 7,8 aufweist, deren Ränder 9,10,11,12 nach innen - einen rechten Winkel bildend - umgebogen sind, daß die nach innen zu liegende Schaumstoffschicht 5 des inneren Schaumstoffringes 4 gegenüber den umgebogenen inneren Rändern 10,12 der Abdeckung teilsweise übersteht und daß die obere Abdeckung 7 einen Durchbruch 13 für die Energiezuleitung aufweist.

Die Magnetspule des Gerätes besteht - vorzugsweise - aus Kupferdraht von 1.2 mm Dicke und weist 200 bis 300 Windungen, vorzugsweise 200 Windungen, auf.

Der die Magnetspule umhüllende Schaumstoffkörper 2 ist - vorzugsweise - ein Schaumstoff auf Basis von Polyphenylenoxid (PPO). Es sind allerdings auch andere Schaumstoffe geeignet, beispielsweise einer auf Basis von Polyurethan.

Der für die Abdeckungen verwendete Kunststoff ist bevorzugt ein Äthylen-Propylen-Copolymerisat.

Die kreisrunde, für die Aufnahme des Penis vorgesehene Öffnung im Zentrum hat zweckmäßig einen Durchmesser von etwa 4 cm. Ein solcher Durchmesser stellt in der Regel sicher, daß der Penis auch im abgeschlafften Zustand an der Innenwand des inneren Schaumstoffringes aufliegt bzw. anliegt und daß dem Penis dadurch ein gewisser Halt gegeben wird.

Mit Anschwellen des Penis wird von diesem auf die Innenwand des inneren Schaumstoffringes ein Druck ausgeübt, wobei der Schaumstoff nachgibt, dabei aber mit dem Penis in einem ständigen, innigen Kontakt bleibt. Der innere Schaumstoffring, der aus 2 Schaumstoffschichten verschiedener Porendichte besteht (wobei die nach innen zu liegende Schicht die größere Porendichte aufweist, d.h. weicher ist), ist in der Lage, den zunehmenden Druck aufzunehmen und wird dabei - dem Druck entsprechend mehr oder weniger zusammengepreßt.

Die Porendichte der beiden Schaumstoffschichten des inneren Schaumstoffringes wird so gewählt, daß sich die Schaumstoffschichten
- dem Druck folgend - bis zu einem gewissen Grade zusammenpressen lassen. Das Gerät ist ansonsten so dimensioniert (der Durchmesser beträgt im allgemeinen etwa 9 cm), daß es mit einer Hand umfaßt bzw. gehalten werden kann. Auf diese Weise bleibt das Gerät bequem handhabbar.
   Der angestrebte und eintretende Erfolg beruht auf der kombinierten Wirkung von 2 Faktoren, zum einen auf dem Einfluß des Magnetfeldes und zum anderen auf dem ständigen innigen Kontakt des Penis mit dem Schaumstoff, der durch
- vom Benutzer selbst auszuführende - Hin- und Herbewegungen des Gerätes noch unterstützt und damit verstärkt werden kann.

Mit Hilfe des erfindungsgemäßen Gerätes werden - etwas vereinfacht ausgedrückt - die Gefäße in den Schwellkörpern geöffnet, sodaß Blut einschießen kann mit der Folge, daß der Penis erigiert.

Das zur Anwendung kommende Magnetwechselfeld hat eine Stärke von bis zu 30 Gauß, vorzugsweise von bis zu 20 Gauß. Das Gerät wird über einen Adapter an das übliche Stromnetz angeschlossen. Zwischengeschaltet wird allerdings noch zwischen Adapter und Gerät ein Steuerungsgerät zur stufenlosen Regulierung der Intensität und Frequenz des Magnetfeldes.

Durch regelmäßige Benutzung des Gerätes gelingt es, die natürlichen, geschlechtsspezifischen physiologischen Funktionen des Mannes bis ins hohe Alter zu erhalten oder da, wo sich bereits ein Nachlassen dieser Funktionen bemerkbar gedacht hat, diese wiederherzustellen, was sich dadurch äußert, daß der Penis seine Erektionsfähigkeit weitgehend wiedererlangt.

Die beigefügten Figuren erläutern die Erfindung.

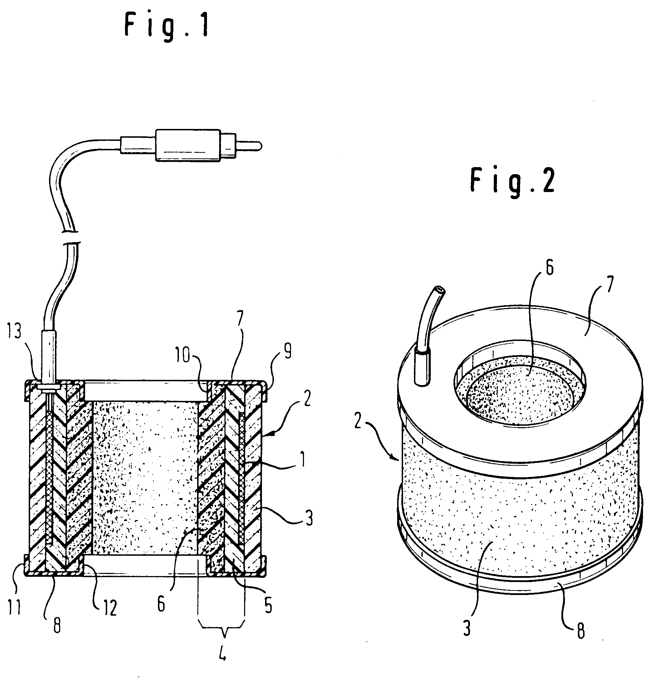

Fig. 1 stellt einen Querschnitt durch das Gerät dar; Fig. 2 ist eine Draufsicht.

In den Figuren bedeuten:
1 Magnetspule
2 Schaumstoffkörper
3 äußerer Schaumstoffring
4 innerer Schaumstoffring
5 innere Schaumstoffschicht von 4
6 zweite Schaumstoffschicht von 4
7 und 8 Abdeckungen
9,10,11,12 Ränder der Abdeckungen
13 Durchbruch mit Energiezuleitung

## Patentansprüche

1. Gerät zur Erhaltung bzw. Wiederherstellung der natürlichen, geschlechtsspezifischen physiologischen Funktionen des Mannes, dad. gek., daß es besteht aus einer ringförmigen Magnetspule (1), die allseitig von einem Schaumstoffkörper (2) umhüllt ist, und zwar von einem - von unten nach oben durchgehenden - äußeren, aus einer Kunststoffschicht mit einheitlicher Porendichte bestehenden Schaumstoffring (3) und von einem - von unten nach oben durchgehenden - inneren, aus 2 Schaumstoffschichten verschiedener Porendichte bestehenden Schaumstoffring (4), bei dem die nach innen zu liegende Schaumstoffschicht (5) gegenüber der zweiten Schaumstoffschicht (6) eine größere Porendichte aufweist, daß der die Magnetspule umhüllende Schaumstoffkörper (2) am unteren und oberen Ende jeweils eine ringförmige kappenartige Abdeckung aus Kunststoff (7,8) aufweist, deren Ränder (9,10,11,12) nach innen - einen rechten Winkel bildend - umgebogen sind, daß die nach innen zu liegende Schaumstoffschicht (5) des inneren Schaumstoffringes (4) gegenüber den umgebogenen inneren Rändern der der Abdeckung (10,12) teilweise übersteht und daß die obere Abdeckung einen Durchbruch (13) für die Energiezuleitung aufweist.

2. Gerät gemäß Anspruch 1, dad. gek., daß der Durchmesser der zur Aufnahme des Penis bestimmten, ringförmigen Öffnung im Zentrum etwa 4 cm beträgt und daß der äußere Durchmesser des Gerätes etwa 9 cm beträgt.

3. Gerät gemäß Anspruch 1 und 2, dad. gek., daß die Magnetspule (1) aus Kupferdraht von 1.2 mm Dicke besteht und 200 bis 300 Windungen, vorzugsweise 200 Windungen, aufweist.

4. Gerät gemäß Anspruch 1 bis 3, dad. gek., daß der Schaumstoff einer auf Basis von Polyphenylenoxid (PPO) ist.

5. Gerät gemaß Anspruch 1 bis 4, dad. gek., daß die Abdeckung aus einem Kunststoff besteht.

6. Gerät gemäß Anspruch 5, dad. gek., daß der Kunststoff ein Äthylen-Propylen-Copolymerisat ist.
